# EUROPEAN PATENT SPECIFICATION

(11) **EP 4 305 643 B1**
(45) Date of publication and mention of the grant of the patent: **11.11.2026**
(21) Application number: 22722016.7
(22) Date of filing: 19.04.2022
(51) Int. Cl.: G16H 50/20

(54) **LEARNING AND PREDICTING TEMPORAL PROFILES OF PHYSIOLOGICAL STATES ASSOCIATED WITH THE ADMINISTRATION OF COMMONLY USED CRITICAL CARE DRUGS**
LERNEN UND VORHERSAGEN VON ZEITLICHEN PROFILEN PHYSIOLOGISCHER ZUSTÄNDE, DIE MIT DER VERABREICHUNG VON GEMEINSAM VERWENDETEN INTENSIVMEDIZINEN ASSOZIIERT SIND
APPRENTISSAGE ET PRÉDICTION DE PROFILS TEMPORELS D'ÉTATS PHYSIOLOGIQUES ASSOCIÉS À L'ADMINISTRATION DE MÉDICAMENTS DE SOINS INTENSIFS COURAMMENT UTILISÉS

(30) Priority: 30.04.2021 US 202163182742 P
(43) Date of publication of application: 17.01.2024
(73) Proprietor: Becton, Dickinson and Company, Franklin Lakes, NJ 07417-1880 (US)
(72) Inventor: POTES BLANDON, Cristhian, M., Irvine, CA 92614 (US); LEE, Christine, Long Beach, CA 90814 (US); MOSES, Kevin, James, Irvine, CA 92614 (US); AL HATIB, Feras, Irvine, CA 92614 (US)
(74) Representative: Venner, Julia Ann
(86) International application number: PCT/US2022/025404
(87) International publication number: WO 2022/231890

(56) References cited:
- WO-A1-2020/245823
- US-A1- 2014 058 273

## Description

### RELATED APPLICATIONS

This application claims priority based on United States Provisional Patent Application Serial No. 63/182,742, filed 30 April 2021 and entitled LEARNING AND PREDICTING TEMPORAL PROFILES OF PHYSIOLOGICAL STATES ASSOCIATED WITH THE ADMINISTRATION OF COMMONLY USED CRITICAL CARE DRUGS.

### BACKGROUND

The present disclosure relates generally to the assessment of physiological states of a critical care patient, and more specifically to physiological states associated with clinical interventions.

Hemodynamic parameters, including blood pressure and heart rate, can be influenced by the administration of fluids and certain drugs, including but not limited to vasopressors, inotropes, and analgesics. Conventional patient monitoring in hospital settings can include continuous or periodic hemodynamic monitoring, which can detect changes in hemodynamic parameters but cannot differentiate hemodynamic changes due to fluid or drug administration from other events that may cause similar hemodynamic changes. For example, nociception, which is the detection of painful stimuli, can cause hemodynamic changes like those caused by the administration of drugs. Because the presence of nociception in critically ill patients may not be observable or communicated, it is desirable to be able to detect or predict nociception events in real time, to assist a clinician in the delivery of care.

Because hemodynamic changes associated with nociception events can be similar to hemodynamic changes associated with the administration of fluids and drugs, it is desirable to develop a technique for differentiating hemodynamic changes caused by the administration of fluids or drugs from nociception events.

US 2014/058273 describes apparatus, systems and methods analyzing pressure and volume waveforms in the vasculature.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

A method for identifying physiological states of a patient, useful for understanding the presently claimed invention, includes performing, by a hemodynamic monitor, waveform analysis of hemodynamic data to determine a plurality of profiling parameters, wherein the hemodynamic data is sensed hemodynamic data representative of an arterial pressure waveform of the patient that has been received by the hemodynamic monitor; extracting, by the hemodynamic monitor, a patient data segment comprising a patient data set for a first profiling parameter of the plurality of profiling parameters; comparing, by the hemodynamic monitor, the patient data segment to a plurality of stored data segments from a database, each of the plurality of stored data segments having an associated stored discrete state data set indicative of whether a clinical intervention was administered and a stored data set for the first profiling parameter; identifying, by the hemodynamic monitor, a plurality of stored data segments satisfying threshold similarity criteria with respect to the patient data segment; and displaying, by the hemodynamic monitor, a predicted discrete state indicator of the patient.

A system for identifying physiological states of a patient and providing an indicator of the identified physiological states to medical personnel, useful for understanding the presently claimed invention, includes a hemodynamic sensor that produces hemodynamic data representative of an arterial pressure waveform of the patient; a system memory that stores physiological state prediction software code and a database; a user interface that displays a predicted discreet state of a patient to the medical personnel; and a hardware processor that is configured to execute the physiological state prediction software code. Executing the physiological state prediction software code includes performing a waveform analysis of the hemodynamic data to determine a plurality of profiling parameters; extracting a patient data segment comprising a patient data set for a first profiling parameter of the plurality of profiling parameters; comparing the patient data segment to a plurality of stored data segments from a look-up table in a database, each of the plurality of stored data segments having an associated discrete state data set indicative of whether a clinical intervention was administered and a data set for the first profiling parameter; and identifying a plurality of stored data segments satisfying threshold similarity criteria with respect to the patient data segment.

A system for training a predictive model to predict a physiological state indicative of whether a clinical intervention was administered based upon a monitored arterial pressure of a patient, useful for understanding the presently claimed invention, includes a system memory that stores model training software code for the predictive model; a hardware processor configured to execute the model training software code to receive hemodynamic data representing an arterial pressure waveform of a critical care patient population and including data labels indicating a type of clinical intervention and approximate time of administration of the clinical intervention; divide the critical care patient population hemodynamic data into a training patient subset population and a validation patient subset population; transform the hemodynamic data to a plurality of profiling parameters characterizing the hemodynamic data; extract a plurality of data segments from hemodynamic data for each patient in the training patient subset population and the validation patient subset population, each data segment of the plurality of data segments representing a fixed time period and comprising (a) data points determined for a profiling parameter over the fixed period of time and (b) the data labels indicating a critical care intervention if administered during the fixed period of time; compare data segments extracted from the validation patient subset population to data segments extracted from the training patient subset population using a difference metric to identify data segments satisfying threshold similarity criteria; and calculate a probability of administration of a critical care intervention for each data segment of the plurality of data segments extracted from the validation patient subset population.

The present summary is provided only by way of example, and not limitation. Other aspects of the present disclosure will be appreciated in view of the entirety of the present disclosure, including the entire text, claims and accompanying figures.

### BRIEF DESCRIPTION OF THE DRAWINGS

FIG. 1 is a perspective view of an example hemodynamic monitor that determines a probability of a physiological state of a patient associated with administration of critical care drugs.
FIG. 2 is a perspective view of an example minimally invasive pressure sensor for sensing hemodynamic data representative of arterial pressure of a patient.
FIG. 3 is a perspective view of an example non-invasive sensor for sensing hemodynamic data representative of arterial pressure of a patient.
FIG. 4 is a graph illustrating an example trace of an arterial pressure waveform from which physiological state profiling parameters can be determined.
FIG. 5 is an example of a data set for profiling parameters determined through waveform analysis and data labels indicating the administration of a clinical intervention.
FIG. 6 is a block diagram illustrating a method for training a predictive model.
FIG. 7 is a block diagram illustrating an example hemodynamic monitoring system that determines a probability of a physiological state of a patient associated with administration of critical care drugs based on a set of physiological state profiling parameters.
FIG. 8 is an example of a data display of the predicted probability of the physiological state of a patent.
FIG. 9 is a flow diagram illustrating example operations of the hemodynamic monitoring system to generate a set of physiological state profiling parameters and determine a probability of a physiological state of a patient associated with administration of critical care drugs.

While the above-identified figures set forth embodiments of the present invention, other embodiments are also contemplated, as noted in the discussion. In all cases, this disclosure presents the invention by way of representation and not limitation. It should be understood that numerous other modifications and embodiments can be devised by those skilled in the art, which fall within the scope of the invention. The figures may not be drawn to scale, and applications and embodiments of the present invention may include features, steps and/or components not specifically shown in the drawings.

### DETAILED DESCRIPTION

As described herein, a hemodynamic monitoring system implements a predictive physiological state model that produces a probability of a physiological state of a patient being associated with the administration of a clinical intervention. The probability is determined based on physiological state profiling parameters indicative of a clinical intervention as determined by comparing the patient's real-time physiological state profiling parameters with physiological state profiling parameters and associated clinical interventions of a plurality of patients in a critical care population, and identifying similar trends in physiological states and, if present, an associated clinical intervention. As used herein, the term "physiological state" refers to a condition of a patient as indicated by one or more hemodynamic parameters or trends in one or more hemodynamic parameters. Hemodynamic parameters characterize hemodynamic data and include, in the presently claimed invention, a systolic blood pressure, diastolic blood pressure, stroke volume, heart rate, respiration, cardiac contractility, mean arterial pressure, baroreflex sensitivity measures, hemodynamic complexity measures, frequency domain hemodynamic features, or, in examples useful for understanding the presently claimed invention, other vital sign parameters. As used herein, "profiling parameters" includes hemodynamic parameters, which are also referred to herein as "features," and can include a single or subset of hemodynamic parameters determined. As used herein, the term "clinical intervention" refers to the administration of a drug or fluid, including commonly administered critical care drugs such as vasopressors, inotropes, and analgesics, which can affect hemodynamic parameters.

According to techniques of this disclosure, the hemodynamic monitoring system can provide real-time hemodynamic parameters as determined from hemodynamic monitoring and a probability that the physiological state of the patient is associated with the administration of a clinical intervention whether or not a clinical intervention has been administered. This information can be used to assist a clinician in identifying, for example, nociception events, which can have associated hemodynamic parameters that mimic hemodynamic parameters associated with the administration of a clinical intervention. When the administration of a clinical intervention is indicated by a high probability value in the absence of the actual administration of a clinical intervention, the clinician can be alerted to the possibility that the physiological state is not consistent with non-intervention and therefore factors outside of the administration of a clinical intervention (e.g., nociception) may be responsible for the observed physiological state. Furthermore, the predicted probability that the physiological state is associated with a clinical intervention can be input into other prediction models, for example, nociception prediction models, which are trained to alert a clinician to nociception events. The disclosed hemodynamic monitoring system can produce a discrete state label indicative of the probability that the physiological state of the patient is related to the administration of a clinical intervention. The discrete state label, which can indicate a type of clinical intervention, for example, a class of a critical care drug administered, or administration of fluid, can be input into other models to further distinguish hemodynamic parameters associated with clinical interventions from hemodynamic parameters associated with other events. While the disclosed system does not definitively identify nociception events, it can be used in conjunction with other techniques and machine learning systems used to predict nociception. For example, by identifying hemodynamic data associated with clinical intervention, the disclosed system can help rule out nociception.

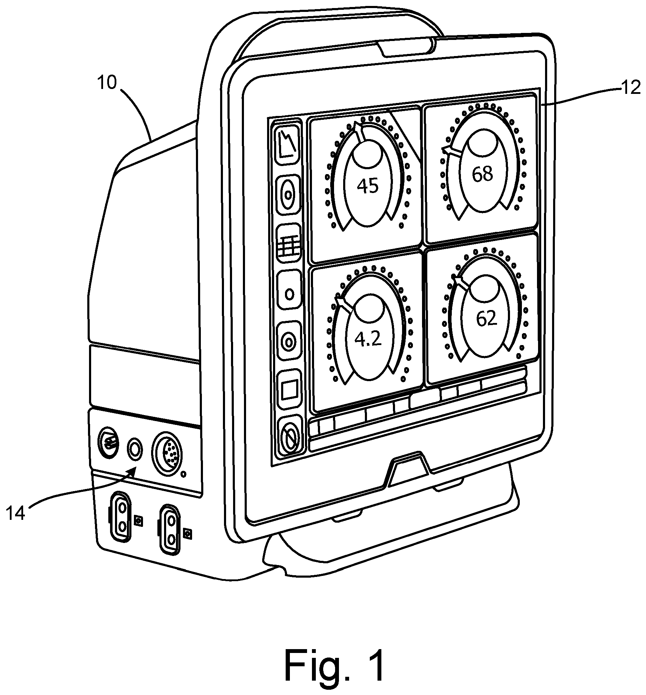

FIG. 1 is a perspective view of hemodynamic monitor 10 that determines a probability of a physiological state associated with the administration of a clinical intervention for a patient. As illustrated in FIG. 1, hemodynamic monitor 10 includes display 12 that, in the example of FIG. 1, presents a graphical user interface including control elements (e.g., graphical control elements) that enable user interaction with hemodynamic monitor 10. Hemodynamic monitor 10 can also include a plurality of input and/or output (I/O) connectors configured for wired connection (e.g., electrical and/or communicative connection) with one or more peripheral components, such as one or more hemodynamic sensors, as is further described below. For instance, as illustrated in FIG. 1, hemodynamic monitor 10 can include I/O connectors 14. While the example of FIG. 1 illustrates five separate I/O connectors 14, it should be understood that in other examples, hemodynamic monitor 10 can include fewer than five I/O connectors or greater than five I/O connectors. In yet other examples, hemodynamic monitor 10 may not include I/O connectors 14, but rather may communicate wirelessly with various peripheral devices.

As is further described below, hemodynamic monitor 10 includes one or more processors and computer-readable memory that stores an arterial pressure waveform analysis software code and physiological state prediction software code. The arterial pressure waveform analysis software code is executable to transform sensed hemodynamic data representative of an arterial pressure waveform of the patient into multiple physiological state profiling parameters (e.g., features), which can include one or more hemodynamic parameters characterizing hemodynamic data of the patient, as well as differential and combinatorial parameters derived from the one or more hemodynamic parameters, as is further described below. The physiological state prediction software code is executable to produce a discrete state label indicative of a probability that the physiological state of the patient is related to the administration of a clinical intervention. For example, hemodynamic monitor 10 can receive sensed hemodynamic data representative of an arterial pressure waveform of the patient, such as via one or more hemodynamic sensors connected to hemodynamic monitor 10 via I/O connectors 14. Hemodynamic monitor 10 executes the arterial pressure waveform analysis software code to obtain, using the received hemodynamic data, multiple physiological state profiling parameters (e.g., features), which can include one or more hemodynamic parameters characterizing hemodynamic data of the patient. Hemodynamic monitor 10 executes the physiological state prediction software code to compare the real-time physiological state profiling parameters of the patient to physiological state profiling parameters collected from a critical care patient population to predict a probability that the patient's physiological state is associated with a clinical intervention.

As described herein, hemodynamic monitor 10 can further utilize patient demographic features and clinical intervention event inputs. Patient demographic features can be used to narrow the critical care population searched to identify similar trends in physiological profiling parameters. As used herein, the term "patient demographic feature" refers to patient characteristics including but not limited to a patient age, age range, gender, disease, or comorbidity. Both patient demographic features and clinical intervention events can be input into hemodynamic monitor 10 by a healthcare worker. Clinical intervention inputs can include, for example, a category, class, or name of a critical care drug or fluid, time of delivery, and delivery rate (e.g., bolus or continuous infusion). Hemodynamic monitor 10 can present graphical control elements (e.g., at a graphical user interface presented at display 12) that enable user input of one or more patient demographic features through inputs received via physical controls (e.g., keypad, or other physical input controls).

For example, as illustrated in FIG. 1, hemodynamic monitor 10 can present a graphical user interface at display 12. Display 12 can be a liquid crystal display (LCD), a light-emitting diode (LED) display, an organic light-emitting diode (OLED) display, or other display device suitable for providing information to users in graphical form. In some examples, such as the example of FIG. 1, display 12 can be a touch-sensitive and/or presence-sensitive display device configured to receive user input in the form of gestures, such as touch gestures, scroll gestures, zoom gestures, swipe gestures, or other gesture input. Hemodynamic monitor 10 presents control elements that enable user input of patient demographic features and clinical intervention events.

In response to received sensed hemodynamic data representative of an arterial pressure waveform of the patient, hemodynamic monitor 10 executes the arterial pressure waveform analysis software code to generate a set of hemodynamic parameters, as is further described below, which can be used as physiological state profiling parameters for determining the probability of association of the physiological state with the administration of a clinical intervention.

Hemodynamic monitor 10 executes the physiological state prediction software code to compare one or more of the hemodynamic parameters, selected as physiological state profiling parameters, to physiological state profiling parameters and associated clinical interventions for a critical care patient population. The physiological state prediction software code identifies physiological state profiling parameters meeting a similarity threshold to produce a predicted probability that the physiological state of the patient is associated with a clinical intervention. The probability of the predicted clinical intervention can be displayed on hemodynamic monitor 10 or can be used in conjunction with a model that requires distinguishing hemodynamic parameters associated with clinical interventions from hemodynamic parameters associated with other causes, such as nociception. For example, a determination by the physiological state prediction software code that one or more hemodynamic parameters are associated with a clinical intervention can effectively silence an alarm for nociception that would otherwise be indicated because of the similarity in the hemodynamic data. As such, the physiological state prediction software code can be used to prevent hemodynamic monitor 10 from alerting a clinician to a nociception event when it is determined that the hemodynamic parameters are consistent with clinical intervention or administration of a critical care drug or fluid.

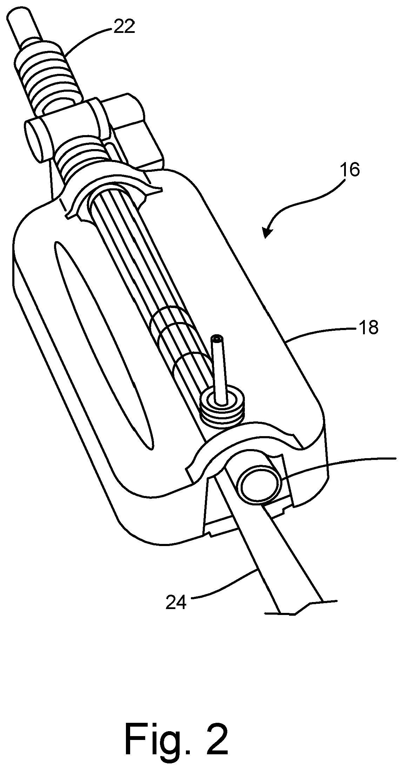

FIG. 2 is a perspective view of hemodynamic sensor 16 that can be attached to a patient for sensing hemodynamic data representative of arterial pressure of the patient. Hemodynamic sensor 16, illustrated in FIG. 2, is one example of a minimally invasive hemodynamic sensor that can be attached to the patient via, e.g., a radial arterial catheter inserted into an arm of the patient. In other examples, hemodynamic sensor 16 can attached to the patient via a femoral arterial catheter inserted into a leg of the patient.

As illustrated in FIG. 2, hemodynamic sensor 16 includes housing 18, fluid input port 20, catheter-side fluid port 22, and I/O cable 24. Fluid input port 20 is configured to be connected via tubing or other hydraulic connection to a fluid source, such as a saline bag or other fluid input source. Catheter-side fluid port 22 is configured to be connected via tubing or other hydraulic connection to a catheter (e.g., a radial arterial catheter or a femoral arterial catheter) that is inserted into an arm of the patient (i.e., a radial arterial catheter) or a leg of the patient (i.e., a femoral arterial catheter). I/O cable 24 is configured to connect to hemodynamic monitor 10 via, e.g., one or more of I/O connectors 14 (FIG. 1). Housing 18 of hemodynamic sensor 16 encloses one or more pressure transducers, communication circuitry, processing circuity, and corresponding electronic components to sense fluid pressure corresponding to arterial pressure of the patient that is transmitted to hemodynamic monitor 10 (FIG. 1) via I/O cable 24.

In operation, a column of fluid (e.g., saline solution) is introduced from a fluid source (e.g., a saline bag) through hemodynamic sensor 16 via fluid input port 20 to catheter-side fluid port 22 toward the catheter inserted into the patient. Arterial pressure is communicated through the fluid column to pressure sensors located within housing 16 which sense the pressure of the fluid column. Hemodynamic sensor 16 translates the sensed pressure of the fluid column to an electrical signal via the pressure transducers and outputs the corresponding electrical signal to hemodynamic monitor 10 (FIG. 1) via I/O cable 24. Hemodynamic sensor 16 therefore transmits analog sensor data (or a digital representation of the analog sensor data) to hemodynamic monitor 10 (FIG. 1) that is representative of substantially continuous beat-to-beat monitoring of the arterial pressure of the patient.

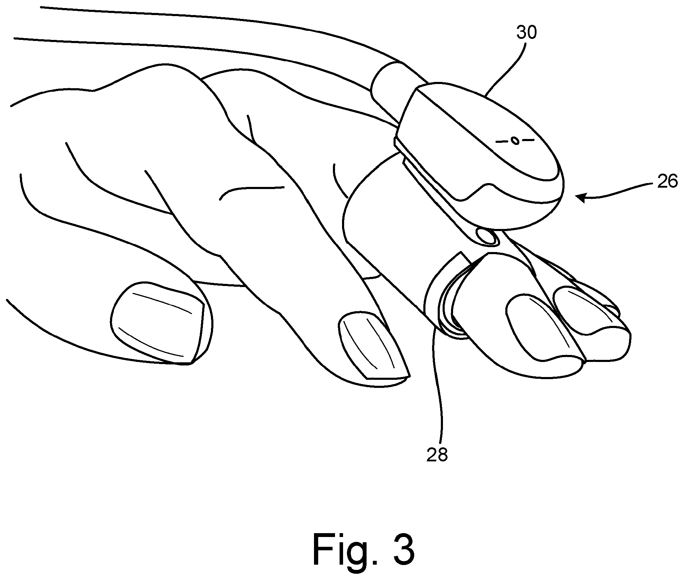

FIG. 3 is a perspective view of hemodynamic sensor 26 for sensing hemodynamic data representative of arterial pressure of a patient. Hemodynamic sensor 26, illustrated in FIG. 3, is one example of a non-invasive hemodynamic sensor that can be attached to the patient via one or more finger cuffs to sense data representative of arterial pressure of the patient. As illustrated in FIG. 3, hemodynamic sensor 26 includes inflatable finger cuff 28 and heart reference sensor 30. Inflatable finger cuff 28 includes an inflatable blood pressure bladder configured to inflate and deflate as controlled by a pressure controller (not illustrated) that is pneumatically connected to inflatable finger cuff 28. Inflatable finger cuff 28 also includes an optical (e.g., infrared) transmitter and an optical receiver that are electrically connected to heart reference sensor 30 to measure the changing volume of the arteries in the finger.

In operation, the pressure controller continually adjusts pressure within the finger cuff to maintain a constant volume of the arteries in the finger (i.e., the unloaded volume of the arteries) as measured by heart reference sensor 30 via the optical transmitter and optical receiver of inflatable finger cuff 28. The pressure applied by the pressure controller to continuously maintain the unloaded volume is representative of the blood pressure in the finger and is communicated by the pressure controller to heart reference sensor 30. Heart reference sensor 30 translates the pressure signal representative of the blood pressure in the finger to hemodynamic data representative of the arterial pressure waveform of the patient, which is transmitted to hemodynamic monitor 10 (FIG. 1) via, e.g., I/O connectors 14 (FIG. 1). Accordingly, hemodynamic sensor 26 transmits sensor data that is representative of substantially continuous beat-to-beat monitoring of the arterial pressure of the patient.

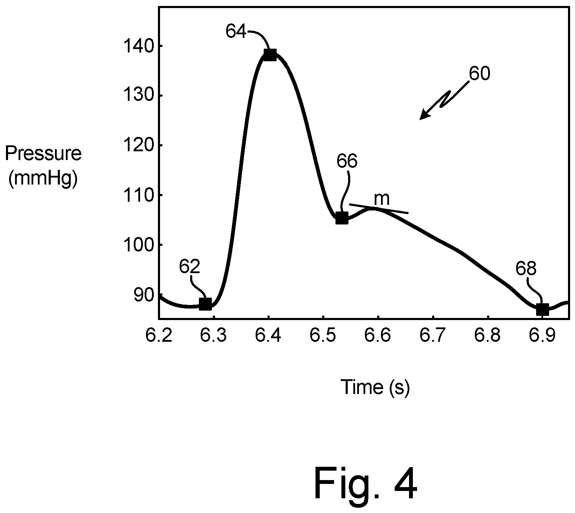

FIG. 4 is a graph illustrating an example trace of arterial pressure waveform 60 corresponding to hemodynamic data sensed by hemodynamic sensor 34 and received by hemodynamic monitor 10. As illustrated in FIG. 4, hemodynamic waveform 60 (e.g., represented via digital hemodynamic data) can include various indicia that can be predictive for a physiological state associated with a clinical intervention for the patient. FIG. 4 illustrates example indicia 62, 64, 66, and 68, corresponding respectively to the start of a heartbeat (indicium 62), the maximum systolic pressure marking the end of systolic rise (indicium 64), the presence of the dicrotic notch marking the end of systolic decay (indicium 66), and the diastole of the heartbeat (indicium 68) of patient 36. Also shown in FIG. 4 is example slope "m" of adjusted arterial pressure waveform 60, though it should be understood that slope "m" is merely representative of multiple slopes that may be determined at multiple locations along arterial pressure waveform 60. Hemodynamic parameters can be extracted from each arterial pressure waveform as known in the art.

### Development of Predictive Physiological State Model

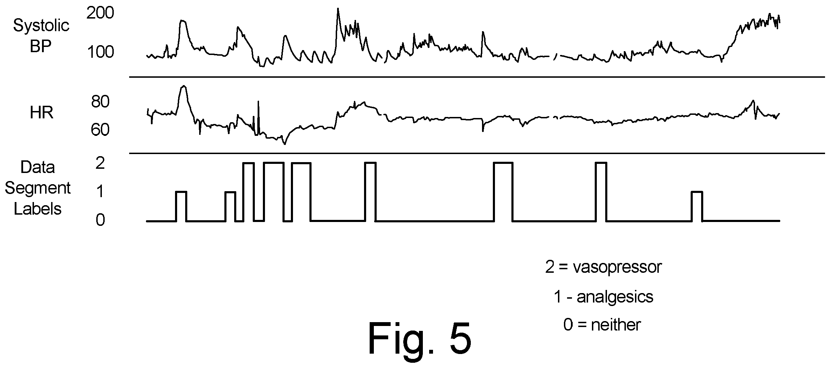

FIG. 5 is an example of a data set for hemodynamic parameters determined through waveform analysis and data labels indicating the administration of a clinical intervention. FIG. 5 illustrates a systolic blood pressure and heart rate over a period of five days derived from the arterial pressure waveform of a patient from a critical care patient population. Data labels indicating the administration of a clinical intervention are provided as values 0, 1, and 2. A value of 2 indicates the administration of a vasopressor, a value of 1 indicates the administration of an analgesic, and a value of 0 indicates no clinical intervention. The type of clinical intervention is not limited to those illustrated in FIG. 5. Other clinical interventions, such as delivery of fluids or the administration of inotropes, among other critical care drugs, can be tracked and documented. Likewise, hemodynamic parameters are not limited to those illustrated in FIG. 5 and can include, for example, stroke volume, heart rate, respiration, cardiac contractility, mean arterial pressure, baroreflex sensitivity measures, hemodynamic complexity measures, and frequency domain hemodynamic features, among others. Data of the type illustrated in FIG. 5 and including additional hemodynamic parameters and patient demographic features is used for training the predictive physiological state model and for creation of a "look up" table, as further described below, for predicting a patient physiological state in a clinical setting.

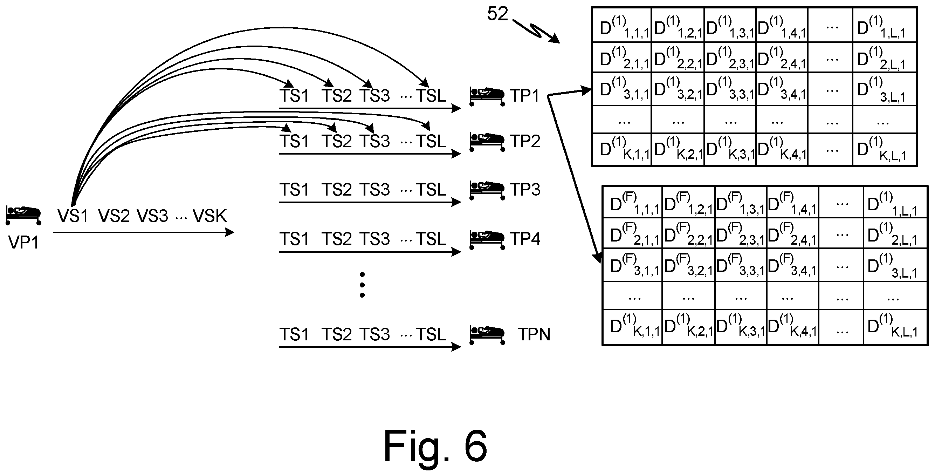

FIG. 6 is a block diagram illustrating a method for training the predictive physiological state model. FIG. 6 shows validation patient VP1, training patients TP1 through TPN where "1" indicates the first patient and "N" indicates the total number of patients, and distance measurement table 54. Validation patient VP1 includes data segments VS1 through VSK where "1" indicates the first data segment and "K" indicates the total number of segments. Each of training patients TP1 though TPN includes data segments TS1 through TSL, where "1" indicates the first data segment and "L" indicates the total number of data segments. The total number of data segments L may or may not equal the total number of validation patient data segments K and can vary across the training patient population such that each patient TP1 through TPN can have a different number of data segments. Each data segment includes data for one or more hemodynamic parameters recorded over a period of time for the associated patient. Each data segment can represent a discrete time interval (e.g., 15 seconds). Data segments can be non-overlapping or can overlap. Data of the type illustrated in FIG. 5 and including data for additional hemodynamic parameters and patient demographic features can be collected for a critical care population having multiple patients. The data can be split by patients into a set of training patients TP1 through TPN and a set of validation patients VP1 through VPM, where "1" indicates the first validation patient and "M" indicates the total number of validation patients. Each validation patient VP1 through VPM has an associated validation data segment VS1 through VSK. The total number of data segments K can vary across the validation patient population such that each validation patient VP1 through VPM can have a different number of data segments.

All hemodynamic data collected for each training patient TP1 through TPN and each validation patient VP1 through VPM can be annotated or labeled with clinical intervention events, as indicated in clinical notes or otherwise provided in electronic medical records. Each training patient TP1 through TPN and each validation patient VP1 through VPM can be further labeled with patient demographic features, including but not limited to patient gender, age, age range, disease, and comorbidities as indicated in electronic medical records.

Hemodynamic data for all patients TP1 through TPN and VP1 through VPM can be divided into discrete segments of time with all segments representing an equal length of time. Each segment can include multiple data points for each hemodynamic parameter collected at defined intervals. For example, a 15-second segment for heart rate data can include 15 heart rate data points collected at 1-second intervals. The segment length can be selected to capture variation in hemodynamic data associated with a clinical intervention event. For example, segments can correspond to a period of time over which no clinical interventions have been administered, a selected period of time preceding a clinical intervention event (e.g., 30 seconds before administration of a clinical intervention), a selected period of time following a clinical intervention event, and a period of time during a clinical intervention event. A segment can include multiple clinical intervention events. For example, a segment can cover a period of time over which multiple types of clinical interventions were administered (e.g., vasopressors and analgesics). A segment can include both a period of time preceding a clinical intervention event and a period of time during the clinical intervention event; or can include both a time during a clinical intervention event and following a clinical intervention event; or can include a period of time preceding a clinical intervention event, a period of time during a clinical intervention event, and a period of time following a clinical intervention event. The administration of a clinical intervention can be relatively short in duration, for example, as provided with the administration of a bolus of a critical care drug, while the effects of the critical care drug on hemodynamic parameters may extend over a longer period of time. Alternatively, the administration of a clinical intervention can be relatively long in duration, for example, as provided with the administration of a continuous infusion of a critical care drug. The length of the segments can be selected and adjusted as needed for training the physiological state predictive model with a goal of identifying trends in hemodynamic data that are indicative of a particular discrete state (i.e., no clinical intervention or a specific type of clinical intervention).

Hemodynamic data for each segment of time can be separated such that each segment TS1 through TSL and VS1 through VSK can include data points for a single hemodynamic parameter. As such, hemodynamic data associated with each validation patient VP1 through VPM and each training patient TP1 through TPN can be divided into segments VS1 through VSK and TS1 through TSL, respectively, for each feature or hemodynamic parameter (e.g., heart rate, systolic blood pressure, etc.). As used herein, the term "feature" can include, in addition to hemodynamic parameters, discrete states (i.e., no clinical intervention or specific type of clinical intervention). It may not be necessary to extract data segments VS1 through VSK and TS1 through TSL for all hemodynamic parameters available for validation patients VP1 through VPM and each training patient TP1 through TPN. Hemodynamic parameters can be selected based on known association with administration of a clinical intervention or known association with other hemodynamic parameters. For example, it is known that the administration of vasopressors can cause an increase in heart rate and systolic blood pressure. Therefore, it is desirable to extract segments VS1 through VSK and TS1 through TSL for both heart rate and systolic blood pressure as each of these hemodynamic parameters may be a valuable indicator of the administration of a clinical intervention.

For each validation patient VP1 through VPM, each data segment VS1 through VSK can be compared to each segment TS1 through TSL for each training patient TP1 through TPN and a distance between each pair of data segments (e.g., VS1 and TS1, VS1 and TS2, VS1 and TS3 for validation patient VP1 and training patient TP1) can be calculated to identify the most similar data segments or data segments that meet a threshold for similarity. For example, as illustrated in FIG. 6, data segment VS1 for validation patient VP1 is compared to all data segments TS1 through TSL for training patient TP1 and is compared to all data segments TS1 through TSL for training patient TP2. This comparison continues for each data segment VS1 through VSK and is applied to each data segment TS1 through TSL for all training patients TP1 through TPN. This comparison is additionally conducted for each feature or hemodynamic parameter selected for analysis.

The distance between each pair of data segments can be calculated using a distance or similarity metric as known in the art. For example, a mean square error distance metric according to the following equation can be used to calculate the distance between each validation patient data segment (VS1 through VSK) and each training patient data segment (TS1 through TSL) for all validation patients VP1 through VPM and all training patients TP1 through TPN: ${D}_{i , j , n}^{\left(f\right)} = {\sum }_{l = 1}^{15} {\left({s}_{i}^{\left(f\right)}\left[l\right]-{s}_{j}^{\left(f\right)}\left[l\right]\right)}^{2}$
wherein D is the distance between a training patient data segment (e.g., TS1) and a validation patient data segment (e.g., VS 1),
f denotes the feature or physiological state profiling parameter (e.g., heart rate, systolic blood pressure, etc.),
*i* denotes the data segment for the validation patient (e.g., VS 1),
*j* denotes the data segment for the training patient (e.g., TS 1),
*n* denotes the training patient (e.g., TP1),
*l* denotes a data collection time point in the data segment (e.g., as illustrated, data points can be collected from *l*=1 second to *l*=15 seconds in 1-second intervals), and
*s* is a value of the profiling parameter determined at time point *l*.

For example, validation patient VP1 may have 400 15-second data segments i, each of which includes a heart rate (*f*) taken at 1-second intervals (*l*). Each of these data segments can be compared to each training patient data segment *j* for each training patient *n* in the training patient population to identify data segments in the training patient population that are most similar to each data segment of validation patient VP1. A distance value of zero indicates equivalency between the pair of segments or highest similarity. All distance values can be recorded in table 52, which as illustrated in FIG. 6, can be subdivided into multiple tables according to validation patient, training patient, and feature. Each square includes a distance measurement between a validation patient segment *i* and a training patient segment *j* shown as:
D^{(f)}_{i, j, n},
where *D* is the distance value,
*f* denotes the feature or physiological state profiling parameter (e.g., heart rate, systolic blood pressure, etc.), ranging from 1 (e.g., heart rate) to F (e.g., systolic blood pressure), wherein each number to F represents a different feature.
*i* identifies the validation patient segment, ranging from 1 to K, wherein each number to K represents a different segment.
*j* identifies the training patient segment, ranging from 1 to L, wherein each number to L represents a different segment,
*n* identifies the training patient, ranging from 1 to N, wherein each number to N represents a different training patient.

For example, square 53 shows a distance calculated between validation patient data segment VS1 and data segment TS1 for training patient TP1 for feature 1.

Distance measurements can be calculated for all data segments for all features for all validation patients and all training patients to identify the most similar segments across all patients. The distance measurements calculated for each feature for a pair of data segments (e.g., VS1 compared to TS1) can added to provide a single distance measurement for that pair of data segments. For example, the distance measurements calculated for heart rate, blood pressure, etc. for VS1 and TS1 for validation patient VP1 and training patient TP1 can be added to provide a single distance measurement for that data segment pair. This can be repeated for all data segment pairs and the values can be ranked in order of lowest to highest, where the lowest value denotes the highest degree of similarity. The most similar data segments (e.g., TP1, TS5; TP5, TS35; TP5, TS135...) can then be identified. The threshold for similarity can be determined and set by the analyst. The threshold for similarity can be a cutoff number of most similar data segments, e.g., in a ranked list in order of similarity, the threshold for similarity can be the most similar 1000 data segments.

The mean square error distant metric described herein is one example of a distance metric that can be used to identify most similar data segments. It will be understood by one of ordinary skill in the art that alternative distance metrics, including but not limited to cosine similarity and Euclidean distance measurement techniques, can be used.

Each of the identified most similar data segments includes an associated discrete state label indicating whether a clinical intervention has been administered and a type of clinical intervention administered at each time point (*l*) in each segment. Comparison of the discrete state labels can be done to determine a probability of a clinical intervention. A probability for clinical intervention can be determined using a statistical model, such as a Markov process or other statistical model as known in the art. A discrete state label associated with the patient hemodynamic data is used to identify whether a clinical intervention has been administered and a type of clinical intervention. For example, all training patients that have received an analgesic is known from the electronic medical records and recorded. Additionally, each administration of an analgesic is tied to one or more data segments indicating the time of administration as provided by the discrete state label. As discussed above, the discrete state can indicate the administration of a particular clinical intervention or no clinical intervention. Within the most similar data set, the number of training patient data segments showing administration of a particular clinical intervention at time 0 (*l*=1 second) is divided by the total number of training patient data segments to determine the initial probability (πᵢ) of the particular clinical intervention. This can be determined for each type of clinical intervention (e.g., vasopressors, fluids, inotropes, etc.). Next, a transition matrix is provided to determine the probability for transitioning from one discrete state to another (e.g., administration of an analgesic to no clinical intervention, or no clinical intervention to administration of a vasopressor, or administration of a vasopressor to administration of an analgesic, etc.) for each data segment within the most similar data set. For example, data segment TS1 may include a discrete state indicating no clinical intervention at time 1 sec through time 7 sec and at time 8 sec, has a discrete state label indicating administration of an analgesic. All data segments within the most similar data set can be analyzed to determine the frequency of transitioning from one discrete state to another discrete state and, specifically, from one particular discrete state to another particular discrete state. For example, for discrete states 0, 1, and 2 illustrated in FIG. 5, a transition matrix includes the number of times the discrete state transitions from 0 to 0, 0 to 1, and 0 to 2, from 1 to 0 and 1 to 1, and 1 to 2, and from 2 to 0, 2 to 1, and 2 to 2. A final probability of a discrete state can be determined by multiplying the initial probability (πᵢ) by the transition matrix as known in the art. Use of the transition matrix can provide further granularity by determining the number of times a transition occurs at each time point in a data segment. For example, how many times a transition from discrete state 0 to discrete state 1 occurs at time *l*=3 seconds. The incorporation of the disclosed transition matrix provides additional information to provide a better estimate of what state a patient is in when the physiological state prediction model is used in a clinical setting.

The physiological state prediction model is developed through a process of iteration in which the predicted physiological state for validation patients VP1 through VPM is compared to actual discrete state labels in the validation patient data segments VS1 through VSK. Multiple parameters can be adjusted and refined through the iteration process to improve the accuracy of the prediction model. For example, the selection of hemodynamic parameters (features), the length of data segment, the similarity threshold for identifying most relevant data segments, the distance metric used to measure distance between data segment pairs, and the transition state calculation for providing increased granularity in characterizing a discrete state of any given segment can all be changed or adjusted to improve the accuracy of prediction, which is indicated by comparing the predicted discrete state for each segment of each validation patient with the actual discrete state as labeled in each validation patient data segment.

The disclosed physiological state prediction model has multiple applications and is not limited to the applications disclosed herein. As further described below, the physiological state prediction model can be used to distinguish hemodynamic data associated with the administration of a critical care drug from hemodynamic data associated with nociception. In another example, the physiological state prediction model can be tailored to predict responsiveness to fluid delivery. The disclosed method could be applied to identify patient segments that are most similar for other physiological states, e.g., hypotensive. For example, a discrete state label could indicate whether a patient is stable or hypotensive.

### Physiological State Prediction in a Clinical Setting

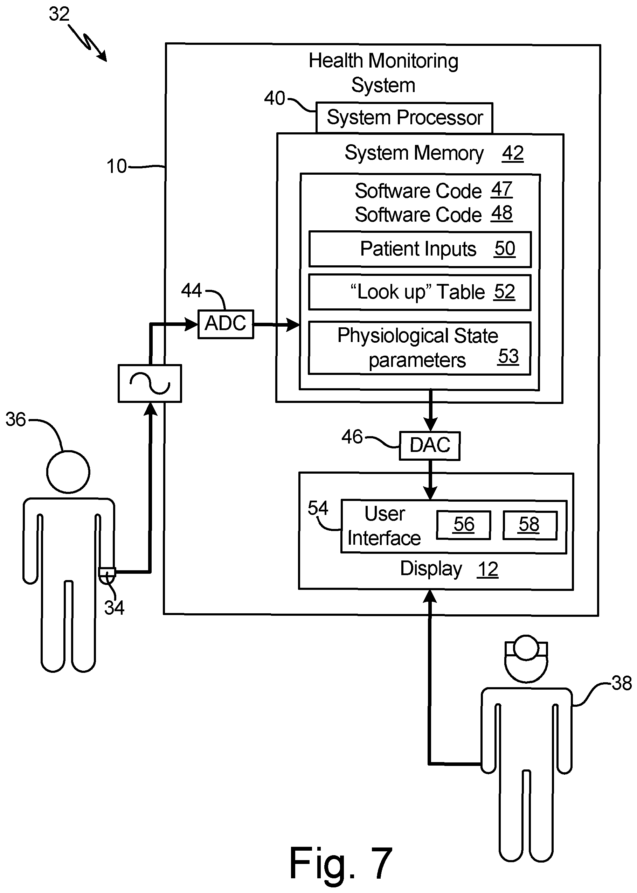

FIG. 7 is a block diagram of hemodynamic monitoring system 32 that determines the probability that the physiological state of the patient is associated with the administration of a clinical intervention based on physiological state profiling parameters, including one or more hemodynamic parameters. As illustrated in FIG. 7, hemodynamic monitoring system 32 includes hemodynamic monitor 10 and hemodynamic sensor 34. Hemodynamic monitoring system 32 can be implemented within a patient care environment, such as an ICU, an OR, or other patient care environment. As illustrated in FIG. 7, the patient care environment can include patient 36 and healthcare worker 38 trained to utilize hemodynamic monitoring system 32.

Hemodynamic monitor 10, as described above with respect to FIG. 1, can be, e.g., an integrated hardware unit including system processor 40, system memory 42, display 12, analog-to-digital (ADC) converter 44, and digital-to-analog (DAC) converter 46. In other examples, any one or more components and/or described functionality of hemodynamic monitor 10 can be distributed among multiple hardware units. For instance, in some examples, display 12 can be a separate display device that is remote from and operatively coupled with hemodynamic monitor 10. In general, though illustrated and described in the example of FIG. 7 as an integrated hardware unit, it should be understood that hemodynamic monitor 10 can include any combination of devices and components that are electrically, communicatively, or otherwise operatively connected to perform functionality attributed herein to hemodynamic monitor 10.

As illustrated in FIG. 7, system memory 42 stores waveform analysis software code 47 and physiological state prediction software code 48. Physiological state prediction software code 48 includes patient inputs 50, "look up" table 52, validation patient and training patient segment data, and patient physiological state profiling parameters 53. Patient inputs 50 can include patient demographic features and clinical intervention events as input by healthcare worker 38. Patient physiological state profiling parameters 53 are determined by waveform analysis software code 27, which is executed to transform sensed hemodynamic data representative of an arterial pressure waveform of the patient. "Look up" table 52 includes all data segments for all training and validation patients and all similarity calculations as illustrated, for example, in table 52 of FIG. 6. Display 12 provides user interface 54, which includes control elements 56 that enable user interaction with hemodynamic monitor 10 and/or other components of hemodynamic monitoring system 32. User interface 54, as illustrated in FIG. 7, can optionally provide sensory alarm 58 to provide warning to medical personnel of a predicted physiological state of patient 36, as is further described below.

Hemodynamic sensor 34 can be attached to patient 36 to sense hemodynamic data representative of an arterial pressure waveform of patient 36. Hemodynamic sensor 34 is operatively connected to hemodynamic monitor 10 (e.g., electrically and/or communicatively connected via wired or wireless connection, or both) to provide the sensed hemodynamic data to hemodynamic monitor 10. In some examples, hemodynamic sensor 34 provides the hemodynamic data representative of the arterial pressure waveform of patient 36 to hemodynamic monitor 10 as an analog signal, which is converted by ADC 44 to digital hemodynamic data representative of the arterial pressure waveform. In other examples, hemodynamic sensor 34 can provide the sensed hemodynamic data to hemodynamic monitor 10 in digital form, in which case hemodynamic monitor 10 may not include or utilize ADC 44. In yet other examples, hemodynamic sensor 34 can provide the hemodynamic data representative of the arterial pressure waveform of patient 36 to hemodynamic monitor 10 as an analog signal, which is analyzed in its analog form by hemodynamic monitor 10.

Hemodynamic sensor 34 can be a non-invasive or minimally invasive sensor attached to patient 36. For instance, hemodynamic sensor 34 can take the form of minimally invasive hemodynamic sensor 16 (FIG. 2), non-invasive hemodynamic sensor 26 (FIG. 3), or other minimally invasive or non-invasive hemodynamic sensor. In some examples, hemodynamic sensor 34 can be attached non-invasively at an extremity of patient 36, such as a wrist, an arm, a finger, an ankle, a toe, or other extremity of patient 36. As such, hemodynamic sensor 34 can take the form of a small, lightweight, and comfortable hemodynamic sensor suitable for extended wear by patient 36 to provide substantially continuous beat-to-beat monitoring of the arterial pressure of patient 36 over an extended period of time, such as minutes or hours.

In certain examples, hemodynamic sensor 34 can be configured to sense an arterial pressure of patient 36 in a minimally invasive manner. For instance, hemodynamic sensor 34 can be attached to patient 36 via a radial arterial catheter inserted into an arm of patient 36. In other examples, hemodynamic sensor 34 can be attached to patient 36 via a femoral arterial catheter inserted into a leg of patient 36. Such minimally invasive techniques can similarly enable hemodynamic sensor 34 to provide substantially continuous beat-to-beat monitoring of the arterial pressure of patient 36 over an extended period of time, such as minutes or hours.

System processor 40 is configured to execute waveform analysis software code 47 and, which provides physiological state profiling parameters 53, including a plurality of hemodynamic parameters. System processor 40 is further configured to execute physiological state prediction software code 48, which determines the probability that the physiological state of patient 36 is associated with the administration of a clinical intervention based on physiological state profiling parameters 53, including one or more hemodynamic parameters, and inputs 50 for patient 36, and "look up" table 52 data for a critical care population. "Look up" table 52 includes all similarity calculations as illustrated, for example, in FIG. 6, as well as all data segments for all training and validation patients. Examples of system processor 40 can include any one or more of a microprocessor, a controller, a digital signal processor (DSP), an application specific integrated circuit (ASIC), a field-programmable gate array (FPGA), or other equivalent discrete or integrated logic circuitry.

System memory 42 can be configured to store information within hemodynamic monitor 10 during operation. System memory 42, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 42 can include volatile and non-volatile computer-readable memories. Examples of volatile memories can include random access memories (RAM), dynamic random access memories (DRAM), static random access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 12 can be a liquid crystal display (LCD), a light-emitting diode (LED) display, an organic light-emitting diode (OLED) display, or other display device suitable for providing information to users in graphical form. User interface 54 can include graphical and/or physical control elements that enable user input to interact with hemodynamic monitor 10 and/or other components of hemodynamic monitoring system 32. In some examples, user interface 54 can take the form of a graphical user interface (GUI) that presents graphical control elements presented at, e.g., a touch-sensitive and/or presence sensitive display screen of display 12. In such examples, user input can be received in the form of gesture input, such as touch gestures, scroll gestures, zoom gestures, or other gesture input. In certain examples, user interface 54 can take the form of and/or include physical control elements, such as a physical buttons, keys, knobs, or other physical control elements configured to receive user input to interact with components of hemodynamic monitoring system 32.

In operation, hemodynamic sensor 34 senses hemodynamic data representative of an arterial pressure waveform of patient 36. Hemodynamic sensor 34 provides the hemodynamic data (e.g., as analog sensor data), to hemodynamic monitor 10. ADC 44 converts the analog hemodynamic data to digital hemodynamic data representative of the arterial pressure waveform of the patient.

System processor 40 receives the arterial pressure waveform from patient 36 and extracts hemodynamic parameters, as well as differential and combinatorial parameters derived from the one or more hemodynamic parameters using waveform analysis software code 47, as provided in the discussion of FIG. 4. System processor 40 can receive patient inputs 50, which can include patient demographic features and clinical intervention events as input via user interface 54. Both patient demographic features and clinical intervention events can be input by healthcare worker 38. Patient demographic features can include, for example, patient age, age range, gender, disease, or comorbidity. Clinical intervention events can include, for example, the administration of a critical care drug or fluid, including time of delivery and/or duration of delivery and dosage.

System processor 40 executes physiological state prediction software code 48 to determine, using the received hemodynamic data, the probability that the physiological state of the patient is associated with the administration of a clinical intervention. System processor 40 can execute physiological state prediction software code 48 to obtain, using the received arterial pressure waveform and extracted hemodynamic parameters, multiple physiological state profiling parameters 53, compare the physiological state profiling parameters 53 to physiological state profiling parameters in "look up" table 52, and determine a probability that the physiological state of the patient is associated with a clinical intervention. Physiological state profiling parameters 52 can include one or more hemodynamic parameters. While multiple hemodynamic parameters can be determined using waveform analysis software code 47, not all hemodynamic parameters determined are necessarily selected as physiological state profiling parameters 53. Physiological state profiling parameters 53 can be selected based on known association with administration of a clinical intervention or known association with other hemodynamic parameters.

Physiological state prediction software code 58 can divide physiological profiling parameters 53 into discrete data segments. Each data segments represents an equal length of time and includes data for one physiological state profiling parameter 53 or feature (e.g., a single hemodynamic parameter). The length of time of each data segment is equal to a length of time of each data segment in "look up" table 52. Data segments can be non-overlapping in time. For example, patient hemodynamic data obtained in real-time can be divided in regular, non-overlapping time intervals, such as every 15 seconds. Additionally, data segments can be started to correspond with a start time of a clinical intervention event, as provided in patient inputs 50. Multiple data segments at each time interval can be extracted from physiological state profiling parameters, with each data segment including data for a single physiological state profiling parameter. For example, discrete data segments can be extracted at each time interval for heart rate, systolic blood pressure, diastolic blood pressure, etc.

Physiological state prediction software code 58 can compare all data segments extracted from physiological state profiling parameters 53 with data segments in "look up" table 52 to identify the most similar "look up" table data segments or "look up" table data segments that meet a threshold for similarity with patient 36. Data segments are compared according to physiological state parameter 53. For example, heart rate data segments from patient 36 are compared only to heart rate data segments in "look up" table 52. Similar data segments can be determined by calculating a distance between pairs of segments using a distance or metric as previously described. For example, a distance between pairs of segments can be determined according to the following equation: ${D}_{i , j , n}^{\left(f\right)} = {\sum }_{l = 1}^{15} {\left({s}_{i}^{\left(f\right)}\left[l\right]-{s}_{j}^{\left(f\right)}\left[l\right]\right)}^{2}$
wherein D is the distance between the "look up" table patient data segment and the data segment extracted from patient 36,
*f* denotes the feature or physiological state profiling parameter (e.g., heart rate, systolic blood pressure, etc.),
*S* is a value of the profiling parameter determined at time point *l* for patient *i* (patient 36) and patient *j* ("look up" table patient), and
*n* is the total number of data points in each data segment.

It is not necessary to compare all data segments extracted from physiological state profiling parameters 53 with data segments in "look up" table 52. The similarity between all data segments in "look up" table 52 is precalculated and provided in "look up" table 52. As such, it is only necessary to find a single "look up" table data segment that is most similar to the patient data segment or meeting the similarity threshold to identify all most similar "look up" table data segments. Inclusion of patient demographic feature can narrow an initial search for most similar data segments. Physiological state prediction software code 48 can first compare patient data segments to "look up" data segments having the same associated demographic features. For example, if patient 36 is a 45 year-old male with diabetes, physiological state prediction software code 48 can first compare the patient data segments with only those data segments in "look up" table 52 that are associated with one or more of the demographic features of the patient, i.e., 45 year-old, male, diabetes. Furthermore, if the patient data segment is associated in time with a clinical intervention, physiological state prediction software code 48 can first compare the patient data segments with only those data segments in "look up" table 52 that are associated with the same clinical intervention.

Once "look up" table data segments meeting the threshold for similarity have been identified, physiological state prediction software code 48 can predict a probability that the physiological state of the patient is associated with a discrete state indicating whether a particular clinical intervention has been administered. The number of "look up" table segments meeting the threshold for similarity is not limited, however, accuracy can be improved with increased data points (i.e., more data segments).

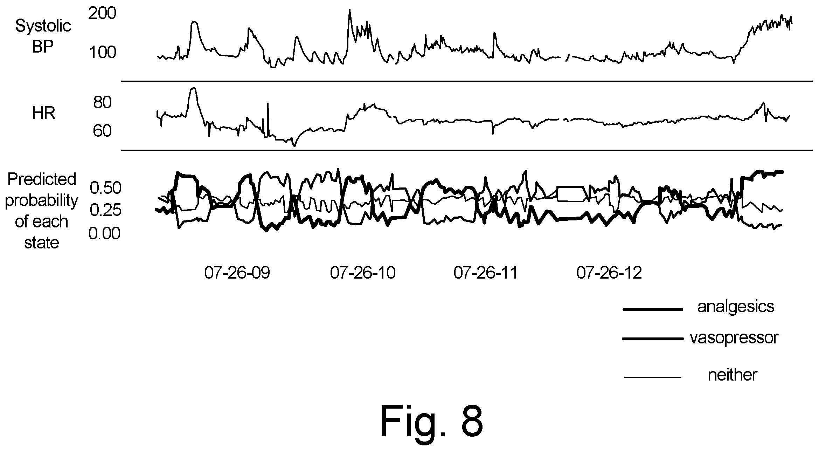

FIG. 8 is an example of a data display on hemodynamic monitor 10 of the predicted probability of the physiological state of patent 36. FIG. 8 shows line graphs of the heart rate and systolic blood pressure of patient 36 as determined in real-time from the arterial pressure waveform of the patient, and a line graph of the probability that the physiological state of the patient is associated with the clinical administration of a vasopressor, analgesic, or neither. Each data point (not identifiable) on the predicted probability graph represents the predicted probability of a physiological state for a single data segment. As illustrated, the sum of the probabilities is equal to 1, such that the probability that any discrete is present can be equal to 0% up to 100%. The data is collected and analyzed in real-time. As such, healthcare worker 38 will be able to recognize when there is a discrepancy between the predicted probability of a discrete state and the actual administration of a clinical intervention or lack thereof as would be administered by the healthcare worker 38.

The graphical display of data illustrated in FIG. 8 is one example of how physiological state data can be presented. In another example, the predicted probability that a physiological state is associated with a clinical intervention can be input into another algorithm developed, for example, for detecting nociception events. For example, the predicted probability can be utilized with an algorithm that predicts a probability of current or future nociception in the patient based on sensed hemodynamic data. The predicted probability can be utilized as a factor in the determination of whether the sensed hemodynamic data indicates a current or future nociception event for the patient. For example, the predicted probability of a current or future nociception event can be inversely proportional to the predicted probability that the physiological state is associated with a clinical intervention. As such, as the predicted probability that the physiological state is associated with a clinical intervention increases, the predicted probability of a nociception event can decrease. As the predicted probability that the physiological state is associated with a clinical intervention decreases, the predicted probability of a nociception event can increase. In other examples, the predicated probability that the physiological state is associated with a clinical intervention can be utilized with an alarm or other notification scheme associated with prediction of a current and/or future nociception event. For instance, a predicted probability that the physiological state is associated with a clinical intervention that satisfies threshold alarming criteria can be utilized to effectively silence (or prevent) invocation of an alarm associated with a prediction of a current and/or future nociception event.

System processor 40 can further execute physiological state prediction software code 48 to invoke sensory alarm 58 via user interface 54 in response to determining, for example, that the physiological state of the patient is associated with nociception. For example, physiological state prediction software code 48 can invoke sensory alarm 58 to warn of a current nociception event. Sensory alarm 58 can be implemented as one or more of a visual alarm, an audible alarm, a haptic alarm, or other type of sensory alarm. For instance, sensory alarm 58 can be invoked as any combination of flashing and/or colored graphics shown by use interface 54 on display 12, display of the risk score via user interface 54 on display 12, a warning sound such as a siren or repeated tone, and a haptic alarm configured to cause hemodynamic monitor 10 to vibrate or otherwise deliver a physical impulse perceptible to healthcare worker 38 or other user.

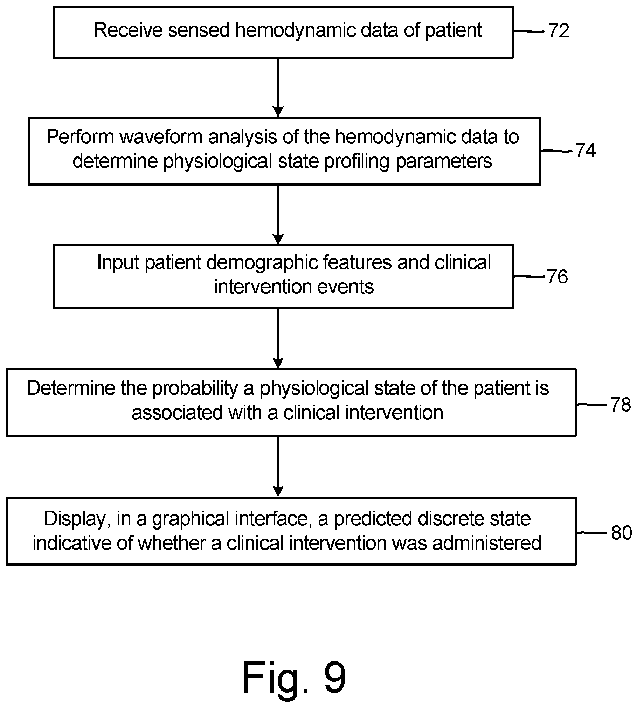

FIG. 9 is a flow diagram illustrating example operations to predict a probability that a physiological state is associated with a clinical intervention. For purposes of clarity and ease of discussion, the example operations are described below within the context of hemodynamic monitoring system 32 of FIG. 4.

Hemodynamic monitor 10 receives sensed hemodynamic data representative of an arterial pressure waveform of patient 36 (Step 72). For instance, hemodynamic monitor 10 can receive an analog hemodynamic sensor signal representative of an arterial pressure waveform of patient 36 from hemodynamic sensor 34.

Hemodynamic monitor 10 performs waveform analysis of the hemodynamic data to determine a plurality of hypotension profiling parameters predictive of a future hypotension event for patient 36 (Step 74). For example, hemodynamic monitor 10 can execute waveform analysis software code 47 to perform waveform analysis of the hemodynamic data to obtain physiological profiling parameters 53 that can be indicative of a clinical intervention. Physiological profiling parameters 53 can include for example, stroke volume, heart rate, respiration, cardiac contractility, mean arterial pressure, baroreflex sensitivity measures, hemodynamic complexity measures, and frequency domain hemodynamic features, among others.

Patient demographic features and clinical intervention events can be input into hemodynamic monitor 10 by healthcare worker 38 (step 76). Patient demographic data can include a patient age, age range, gender, disease, or comorbidity. Clinical intervention events can include the administration of a critical care drug or fluid.

Physiological state prediction software code 48 segregates physiological state profiling parameters into discrete data segments, compares the data segments with data segments in a "look up" table in system memory 42, and predicts a probability that the physiological state of patient 36 is associated with the administration of a clinical intervention (step 78). Physiological state prediction software code 58 can compare all data segments extracted from physiological state profiling parameters 53 with data segments in "look up" table 52 to identify the most similar "look up" table data segments or "look up" table data segments that meet a threshold for similarity with patient 36. Physiological state prediction software code 48 can first compare the patient data segments with only those data segments in "look up" table 52 that are associated with one or more of the demographic features of the patient, e.g., age, gender, comorbidity, or the administration of a clinical intervention to improve searching. Data segments can be compared using a distance metric as known in the art.

Hemodynamic monitor 10 displays the predicted probability that the physiological state of patent 36 is associated with a clinical intervention or, for example, the presence of nociception (step 80).

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

Any relative terms or terms of degree used herein, such as "substantially", "essentially", "generally", "approximately" and the like, should be interpreted in accordance with and subject to any applicable definitions or limits expressly stated herein. In all instances, any relative terms or terms of degree used herein should be interpreted to broadly encompass any relevant disclosed embodiments as well as such ranges or variations as would be understood by a person of ordinary skill in the art in view of the entirety of the present disclosure, such as to encompass ordinary manufacturing tolerance variations, incidental alignment variations, transient alignment or shape variations induced by thermal, rotational or vibrational operational conditions, and the like. Moreover, any relative terms or terms of degree used herein should be interpreted to encompass a range that expressly includes the designated quality, characteristic, parameter or value, without variation, as if no qualifying relative term or term of degree were utilized in the given disclosure or recitation.

## Claims

1. A method for identifying physiological states of a patient and determining a probability that the physiological state of the patient is associated with a clinical intervention, the method comprising:
performing, by a hemodynamic monitor, waveform analysis of hemodynamic data to determine a plurality of profiling parameters, wherein the profiling parameters include one or more of a systolic blood pressure, diastolic blood pressure, standard mean arterial pressure, stroke volume, heart rate, respiration, cardiac contractibility, baroreflex sensitivity measures, hemodynamic complexity measures and frequency domain hemodynamic features, wherein the hemodynamic data is sensed hemodynamic data representative of an arterial pressure waveform of the patient that has been received by the hemodynamic monitor;
extracting, by the hemodynamic monitor, a patient data segment comprising a patient data set for a first profiling parameter of the plurality of profiling parameters;
comparing, by the hemodynamic monitor, the patient data segment to a plurality of stored data segments from a database, each of the plurality of stored data segments having an associated stored discrete state data set which indicates, for each time point of the stored data segment, whether a clinical intervention was administered and a type of clinical intervention if administered, and a stored data set for the first profiling parameter;
identifying, by the hemodynamic monitor, a plurality of stored data segments satisfying threshold similarity criteria with respect to the patient data segment; and
displaying, by the hemodynamic monitor, a predicted discrete state indicator of the patient, wherein the predicted discrete state indicator is a probability of administration of a clinical intervention for the plurality of stored data segments satisfying threshold similarity criteria with respect to the patient data segment.

2. The method of claim 1, wherein the patient data segment and the stored data segment each comprise a fixed time period over which the first profiling parameter is determined for a plurality of time points taken at regular intervals to produce the patient data set for the first profiling parameter and the stored data set for the first profiling parameter.

3. The method of claim 1 or claim 2, wherein the patient data segment further comprises an associated discrete state indicative of whether a clinical intervention was administered during the patient data segment.

4. The method of any preceding claim, wherein the plurality of stored data segments satisfying threshold similarity criteria with respect to the patient data segment are identified by calculating a distance between the patient data segment and each of the plurality of stored data segments using a distance metric.

5. The method of any preceding claim, wherein the plurality of stored data segments comprise data segments obtained from a plurality of patients.

6. The method of claim 5, and further comprising inputting into the hemodynamic monitor a patient classifier, wherein the patient classifier includes a patient age, age range, gender, disease, or comorbidity,
optionally wherein each of the plurality of stored data segments has an associated stored patient classifier matching the patient classifier input into the hemodynamic monitor.

7. A system (32) for identifying physiological states of a patient (36), determining a probability that the physiological state of the patient is associated with a clinical intervention and providing an indicator of the identified physiological states to medical personnel, the system comprising:
a hemodynamic sensor (34) that produces hemodynamic data representative of an arterial pressure waveform (60) of the patient;
a system memory (42) that stores physiological state prediction software code and a database;
a user interface (54) that displays a predicted discreet state indicator of a patient to the
medical personnel, wherein the predicted discrete state indicator is a probability of administration of a clinical intervention for the plurality of stored data segments satisfying threshold similarity criteria with respect to the patient data segment; and
a hardware processor (40) that is configured to execute the physiological state prediction
software code to:
perform a waveform analysis of the hemodynamic data to determine a plurality of profiling parameters (53), wherein the profiling parameters include one or more of a systolic blood pressure, diastolic blood pressure, standard mean arterial pressure, stroke volume, heart rate, respiration, cardiac contractibility, baroreflex sensitivity measures, hemodynamic complexity measures and frequency domain hemodynamic features;
extract a patient data segment comprising a patient data set for a first profiling parameter of the plurality of profiling parameters;
compare the patient data segment to a plurality of stored data segments from a look-up table (52) in a database, each of the plurality of stored data segments having an associated discrete state data set which indicates, for each time point of the stored data segment, whether a clinical intervention was administered and a type of clinical intervention if administered, and a data set for the first profiling parameter; and
identify a plurality of stored data segments satisfying threshold similarity criteria with respect to the patient data segment.

8. The system of claim 7, wherein the patient data segment and the stored data segment each comprise a fixed time period over which the first profiling parameter is determined for a plurality of time points taken at regular intervals to produce the patient data set for the first profiling parameter and the stored data set for the first profiling parameter.

9. The system of claim 7 or claim 8, wherein the patient data segment further comprises an associated discrete state indicative of whether a clinical intervention was administered during the patient data segment.

10. The system of any preceding claim, wherein the plurality of stored data segments satisfying threshold similarity criteria with respect to the patient data segment are identified by calculating a distance between the patient data segment and each of the plurality of stored data segments using a distance metric.

11. The system of any preceding claim, wherein the plurality of stored data segments comprise data segments obtained from a plurality of patients.

12. The system of claim 11, and wherein the database includes a patient classifier, wherein the patient classifier includes an age, age range, gender, disease, or comorbidity of the patient,
optionally wherein each stored data segment of the plurality of stored data segments has an associated stored patient classifier.

13. A system for training a predictive model to predict a physiological state indicative of whether a clinical intervention was administered based upon a monitored arterial pressure of a patient, the system comprising:
a system memory that stores model training software code for the predictive model;
a hardware processor configured to execute the model training software code to:
receive hemodynamic data representing an arterial pressure
waveform of a critical care patient population and including data labels indicating a type of clinical intervention and approximate time of administration of the clinical intervention;
divide the critical care patient population hemodynamic data into:
a training patient subset population; and
a validation patient subset population;
transform the hemodynamic data to a plurality of profiling
parameters characterizing the hemodynamic data, wherein the profiling parameters include one or more of a systolic blood pressure, diastolic blood pressure, standard mean arterial pressure, stroke volume, heart rate, respiration, cardiac contractibility, baroreflex sensitivity measures, hemodynamic complexity measures and frequency domain hemodynamic features;
extract a plurality of data segments from hemodynamic data for each patient in the training patient subset population and the validation patient subset population, each data segment of the plurality of data segments representing a fixed time period and comprising (a) data points determined for a profiling parameter over the fixed period of time and (b) the data labels indicating a critical care intervention if administered during the fixed period of time;
compare data segments extracted from the validation patient subset population to data segments extracted from the training patient subset population using a difference metric to identify data segments satisfying threshold similarity criteria; and
calculate a probability of administration of a critical care intervention for each data segment of the plurality of data segments extracted from the validation patient subset population.

14. The system of claim 13, wherein the probability of administration of a critical care intervention is calculated by determining an (a) initial probability for the administration one or more critical care interventions for the similar data segments, and (b) a probability for transitioning from:
one critical care intervention to another critical care intervention different in type;
no critical care intervention to a critical care intervention; or
a critical care intervention to no critical care intervention.

15. The system of claim 14, wherein:
a) each data segment extracted from the validation patient subset population is compared to each data segment extracted from the training patient subset population for each patient in the training patient subset population and each patient in the validation patient subset population; or
b) the hardware processor is further configured compare the calculated probability of administration of a critical care intervention for each data segment of the plurality of data segments extracted from the validation patient subset population with the data labels included in each data segment.

## Patentansprüche

1. Verfahren zum Identifizieren physiologischer Zustände eines Patienten und Bestimmen einer Wahrscheinlichkeit, dass der physiologische Zustand des Patienten mit einer klinischen Intervention assoziiert ist, wobei das Verfahren Folgendes umfasst:
Durchführen, durch einen hämodynamischen Monitor, einer Wellenformanalyse von hämodynamischen Daten zur Bestimmung einer Vielzahl von Profilierungsparametern, wobei die Profilierungsparameter einen oder mehrere der folgenden Werte umfassen: systolischen Blutdruck, diastolischen Blutdruck, standardisierten mittleren arteriellen Druck, Schlagvolumen, Herzfrequenz, Atmung, Herzkontraktilität, Maße der Baroreflexempfindlichkeit, Maße für die hämodynamische Komplexität sowie hämodynamische Merkmale im Frequenzbereich, wobei es sich bei den hämodynamischen Daten um erfasste hämodynamische Daten handelt, die eine arterielle Druckwellenform des Patienten repräsentieren und vom hämodynamischen Monitor empfangen wurden;
Extrahieren, durch den hämodynamischen Monitor, eines Patientendatensegments, das einen Patientendatensatz für einen ersten Profilierungsparameter der Vielzahl von Profilierungsparametern umfasst;
Vergleichen, durch den hämodynamischen Monitor, des Patientendatensegments mit einer Vielzahl von gespeicherten Datensegmenten aus einer Datenbank, wobei jedes der Vielzahl von gespeicherten Datensegmenten einen assoziierten gespeicherten Datensatz mit diskretem Zustand aufweist, der für jeden Zeitpunkt des gespeicherten Datensegments angibt, ob ein klinischer Eingriff verabreicht wurde und eine Art von klinischem Eingriff, falls verabreicht, und einen gespeicherten Datensatz für den ersten Profilierungsparameter;
Identifizieren, durch den hämodynamischen Monitor, einer Vielzahl von gespeicherten Datensegmenten, die Schwellenähnlichkeitskriterien in Bezug auf das Patientendatensegment erfüllen; und
Anzeigen, durch den hämodynamischen Monitor, eines vorhergesagten diskreten Zustandsindikators des Patienten, wobei der vorhergesagte diskrete Zustandsindikator eine Wahrscheinlichkeit einer Verabreichung eines klinischen Eingriffs für die Vielzahl von gespeicherten Datensegmenten ist, die Schwellenähnlichkeitskriterien in Bezug auf das Patientendatensegment erfüllen.

2. Verfahren nach Anspruch 1, wobei das Patientendatensegment und das gespeicherte Datensegment jeweils einen festen Zeitraum umfassen, über den der erste Profilierungsparameter für eine Vielzahl von Zeitpunkten bestimmt wird, die in regelmäßigen Intervallen genommen werden, um den Patientendatensatz für den ersten Profilierungsparameter und den gespeicherten Datensatz für den ersten Profilierungsparameter zu erzeugen.

3. Verfahren nach Anspruch 1 oder Anspruch 2, wobei das Patientendatensegment ferner einen assoziierten diskreten Zustand umfasst, der angibt, ob eine klinische Intervention während des Patientendatensegments verabreicht wurde.

4. Verfahren nach einem der vorhergehenden Ansprüche, wobei die Vielzahl von gespeicherten Datensegmenten, die Schwellenähnlichkeitskriterien in Bezug auf das Patientendatensegment erfüllen, durch Berechnen eines Abstands zwischen dem Patientendatensegment und jedem der Vielzahl von gespeicherten Datensegmenten unter Verwendung einer Abstandsmetrik identifiziert werden.

5. Verfahren nach einem der vorhergehenden Ansprüche, wobei die Vielzahl von gespeicherten Datensegmenten Datensegmente umfassen, die von einer Vielzahl von Patienten erhalten werden.

6. Verfahren nach Anspruch 5, das ferner das Eingeben eines Patientenklassifikators in den hämodynamischen Monitor umfasst, wobei der Patientenklassifikator ein Patientenalter, einen Altersbereich, ein Geschlecht, eine Erkrankung oder eine Komorbidität umfasst,
wobei optional jedes der Vielzahl von gespeicherten Datensegmenten einen assoziierten gespeicherten Patientenklassifikator aufweist, der mit dem Patientenklassifikator übereinstimmt, der in den hämodynamischen Monitor eingegeben wird.

7. System (32) zum Identifizieren physiologischer Zustände eines Patienten (36), Bestimmen einer Wahrscheinlichkeit, dass der physiologische Zustand des Patienten mit einer klinischen Intervention assoziiert ist, und Bereitstellen eines Indikators der identifizierten physiologischen Zustände für medizinisches Personal, wobei das System Folgendes umfasst:
einen hämodynamischen Sensor (34), der hämodynamische Daten erzeugt, die eine arterielle Druckwellenform (60) des Patienten repräsentieren;
einen Systemspeicher (42), der einen Softwarecode zur Vorhersage physiologischer Zustände und eine Datenbank speichert;
eine Benutzerschnittstelle (54), die dem medizinischen Personal einen vorhergesagten diskreten Zustandsindikator eines Patienten anzeigt, wobei der vorhergesagte diskrete Zustandsindikator eine Wahrscheinlichkeit einer Verabreichung eines klinischen Eingriffs für die Vielzahl von gespeicherten Datensegmenten ist, die Schwellenähnlichkeitskriterien in Bezug auf das Patientendatensegment erfüllen; und
einen Hardwareprozessor (40), der dazu ausgelegt ist, den physiologischen Zustandsvorhersage-Softwarecode für Folgendes auszuführen:
Durchführen einer Wellenformanalyse der hämodynamischen Daten, um eine Vielzahl von Profilierungsparametern (53) zu bestimmen,
wobei die Profilierungsparameter einen oder mehrere der folgenden Werte umfassen: systolischen Blutdruck, diastolischen Blutdruck, standardisierten mittleren arteriellen Druck, Schlagvolumen, Herzfrequenz, Atmung, Herzkontraktilität, Messgrößen der Baroreflexempfindlichkeit, Messgrößen der hämodynamischen Komplexität und hämodynamische Merkmale im Frequenzbereich;
Extrahieren eines Patientendatensegments, das einen Patientendatensatz für einen ersten Profilierungsparameter der Vielzahl von Profilierungsparametern umfasst;
Vergleichen des Patientendatensegments mit einer Vielzahl von gespeicherten Datensegmenten aus einer Nachschlagetabelle (52) in einer Datenbank, wobei jedes der Vielzahl von gespeicherten Datensegmenten einen assoziierten diskreten Zustandsdatensatz aufweist, der für jeden Zeitpunkt des gespeicherten Datensegments angibt, ob ein klinischer Eingriff verabreicht wurde und eine Art von klinischem Eingriff, falls verabreicht, und einen Datensatz für den ersten Profilierungsparameter; und
Identifizieren einer Vielzahl von gespeicherten Datensegmenten, die Schwellenähnlichkeitskriterien in Bezug auf das Patientendatensegment erfüllen.

8. System nach Anspruch 7, wobei das Patientendatensegment und das gespeicherte Datensegment jeweils einen festen Zeitraum umfassen, über den der erste Profilierungsparameter für eine Vielzahl von Zeitpunkten bestimmt wird, die in regelmäßigen Intervallen genommen werden, um den Patientendatensatz für den ersten Profilierungsparameter und den gespeicherten Datensatz für den ersten Profilierungsparameter zu erzeugen.

9. System nach Anspruch 7 oder Anspruch 8, wobei das Patientendatensegment ferner einen assoziierten diskreten Zustand umfasst, der angibt, ob eine klinische Intervention während des Patientendatensegments verabreicht wurde.

10. System nach einem der vorhergehenden Ansprüche, wobei die Vielzahl von gespeicherten Datensegmenten, die Schwellenähnlichkeitskriterien in Bezug auf das Patientendatensegment erfüllen, durch Berechnen eines Abstands zwischen dem Patientendatensegment und jedem der Vielzahl von gespeicherten Datensegmenten unter Verwendung einer Abstandsmetrik identifiziert werden.

11. System nach einem der vorhergehenden Ansprüche, wobei die Vielzahl von gespeicherten Datensegmenten Datensegmente umfassen, die von einer Vielzahl von Patienten erhalten werden.

12. System nach Anspruch 11, und wobei die Datenbank einen Patientenklassifikator beinhaltet, wobei der Patientenklassifikator ein Alter, einen Altersbereich, ein Geschlecht, eine Krankheit oder eine Komorbidität des Patienten beinhaltet,
wobei optional jedes gespeicherte Datensegment der Vielzahl von gespeicherten Datensegmenten einen assoziierten gespeicherten Patientenklassifikator aufweist.

13. System zum Trainieren eines Vorhersagemodells zum Vorhersagen eines physiologischen Zustands, der angibt, ob eine klinische Intervention basierend auf einem überwachten arteriellen Druck eines Patienten verabreicht wurde, wobei das System Folgendes umfasst:
einen Systemspeicher, der Modelltrainings-Softwarecode für das prädiktive Modell speichert;
einen Hardwareprozessor, der dazu ausgelegt ist, den Modelltrainings-Softwarecode für Folgendes auszuführen:
Empfangen von hämodynamischen Daten, die eine arterielle Druckwellenform einer Intensivpatientenpopulation repräsentieren und Datenmarkierungen beinhalten, die eine Art klinischer Intervention und eine ungefähre Verabreichungszeit der klinischen Intervention angeben;
Unterteilen der hämodynamischen Daten der Intensivpatientenpopulation in:
eine Trainingspatienten-Teilpopulation; und
eine Validierungspatienten-Teilpopulation;
Umwandeln der hämodynamischen Daten in eine Vielzahl von Profilierungsparametern, die die hämodynamischen Daten charakterisieren, wobei die Profilierungsparameter einen oder mehrere der folgenden Werte umfassen: systolischen Blutdruck, diastolischen Blutdruck, standardisierten mittleren arteriellen Druck, Schlagvolumen, Herzfrequenz, Atmung, Herzkontraktilität, Maße der Baroreflexempfindlichkeit, Maße der hämodynamischen Komplexität und hämodynamische Merkmale im Frequenzbereich;
Extrahieren einer Vielzahl von Datensegmenten aus hämodynamischen Daten für jeden Patienten in der Trainingspatienten-Teilpopulation und der Validierungspatienten-Teilpopulation, wobei jedes Datensegment der Vielzahl von Datensegmenten einen festen Zeitraum repräsentiert und Folgendes umfasst: (a) Datenpunkte, die für einen Profilerstellungsparameter über den festen Zeitraum bestimmt sind, und (b) die Datenmarkierungen, die eine intensivmedizinische Maßnahme angeben, wenn sie während der festen Zeitperiode verabreicht wird;
Vergleichen von Datensegmenten, die aus der Validierungspatienten-Teilpopulation extrahiert wurden, mit Datensegmenten, die aus der Trainingspatienten-Teilpopulation extrahiert wurden, unter Verwendung einer Differenzmetrik, um Datensegmente zu identifizieren, die Schwellenähnlichkeitskriterien erfüllen; und
Berechnen einer Wahrscheinlichkeit der Verabreichung einer intensivmedizinischen Maßnahme für jedes Datensegment der Vielzahl von Datensegmenten, die aus der Validierungspatienten-Teilpopulation extrahiert wurden.

14. System nach Anspruch 13, wobei die Wahrscheinlichkeit der Durchführung einer intensivmedizinischen Maßnahme berechnet wird, indem Folgendes bestimmt wird: (a) eine anfängliche Wahrscheinlichkeit für die Durchführung einer oder mehrerer intensivmedizinischer Maßnahmen für die ähnlichen Datensegmente, und (b) eine Wahrscheinlichkeit für den Übergang von:
einer intensivmedizinischen Maßnahme zu einer anderen intensivmedizinischen Maßnahme, die sich je nach Art unterscheidet;
keiner intensivmedizinischen Maßnahme zu einer intensivmedizinischen Maßnahme; oder
einer intensivmedizinischen Maßnahme zu keiner intensivmedizinischen Maßnahme.

15. System nach Anspruch 14, wobei:
a) jedes aus der Validierungspatienten-Teilpopulation extrahierte Datensegment mit jedem aus der Trainingspatienten-Teilpopulation extrahierten Datensegment verglichen wird, und zwar für jeden Patienten in der Trainingspatienten-Teilpopulation und jeden Patienten in der Validierungspatienten-Teilpopulation; oder
b) der Hardwareprozessor ferner dazu ausgelegt ist, die berechnete Wahrscheinlichkeit der Verwaltung einer intensivmedizinischen Maßnahme für jedes Datensegment der Vielzahl von Datensegmenten, die aus der Validierungspatienten-Teilpopulation extrahiert wurden, mit den in jedem Datensegment enthaltenen Datenmarkierungen zu vergleichen.

## Revendications

1. Procédé d'identification d'états physiologiques d'un patient et de détermination d'une probabilité que l'état physiologique du patient soit associé à une intervention clinique, le procédé comprenant :
la réalisation, par un moniteur hémodynamique, d'une analyse de forme d'onde de données hémodynamiques afin de déterminer une pluralité de paramètres de profilage,
les paramètres de profilage comportant un ou plusieurs paramètres parmi une pression artérielle systolique, une pression artérielle diastolique, une pression artérielle moyenne standard, un volume d'éjection systolique, une fréquence cardiaque, une respiration, une contractilité cardiaque, des mesures de sensibilité baroréflexe, des mesures de complexité hémodynamique et des caractéristiques hémodynamiques dans le domaine fréquentiel, les données hémodynamiques étant des données hémodynamiques détectées représentant une forme d'onde de pression artérielle du patient qui ont été reçues par le moniteur hémodynamique ;
l'extraction, par le moniteur hémodynamique, d'un segment de données patient comprenant un ensemble de données patient pour un premier paramètre de profilage de la pluralité de paramètres de profilage ;
la comparaison, par le moniteur hémodynamique, du segment de données patient à une pluralité de segments de données stockés provenant d'une base de données, chacun de la pluralité de segments de données stockés présentant un ensemble de données d'état discret stocké associé qui indique, pour chaque point temporel du segment de données stocké, si une intervention clinique a été ou non administrée et un type d'intervention clinique si elle a été administrée, et un ensemble de données stocké pour le premier paramètre de profilage ;
l'identification, par le moniteur hémodynamique, d'une pluralité de segments de données stockés satisfaisant à des critères de similarité à seuil par rapport au segment de données patient ; et
l'affichage, par le moniteur hémodynamique, d'un indicateur d'état discret prédit du patient, l'indicateur d'état discret prédit étant une probabilité d'administration d'une intervention clinique pour la pluralité de segments de données stockés satisfaisant à des critères de similarité à seuil par rapport au segment de données patient.

2. Procédé selon la revendication 1, dans lequel le segment de données patient et le segment de données stocké comprennent chacun une période de temps fixe sur laquelle le premier paramètre de profilage est déterminé pour une pluralité de points temporels pris à intervalles réguliers afin de produire l'ensemble de données patient pour le premier paramètre de profilage et l'ensemble de données stocké pour le premier paramètre de profilage.

3. Procédé selon la revendication 1 ou la revendication 2, dans lequel le segment de données patient comprend en outre un état discret associé indiquant si une intervention clinique a été ou non administrée pendant le segment de données patient.

4. Procédé selon l'une quelconque des revendications précédentes, dans lequel la pluralité de segments de données stockés satisfaisant à des critères de similarité à seuil par rapport au segment de données patient sont identifiés par calcul d'une distance entre le segment de données patient et chacun de la pluralité de segments de données stockés au moyen d'une métrique de distance.

5. Procédé selon l'une quelconque des revendications précédentes, dans lequel la pluralité de segments de données stockés comprennent des segments de données obtenus à partir d'une pluralité de patients.

6. Procédé selon la revendication 5, et comprenant en outre l'application, en entrée du moniteur hémodynamique, d'un classificateur de patient, le classificateur de patient comportant l'âge du patient, une tranche d'âge, le sexe, une maladie ou une comorbidité, facultativement, dans lequel chacun de la pluralité de segments de données stockés présente un classificateur de patient stocké associé qui correspond au classificateur de patient appliqué en entrée du moniteur hémodynamique.

7. Système (32) d'identification d'états physiologiques d'un patient (36), de détermination d'une probabilité que l'état physiologique du patient soit associé à une intervention clinique et de fourniture à un personnel médical d'un indicateur des états physiologiques identifiés, le système comprenant :
un capteur hémodynamique (34) qui produit des données hémodynamiques représentant une forme d'onde de pression artérielle (60) du patient ;
une mémoire système (42) qui stocke un code logiciel de prédiction d'état physiologique et une base de données ;
une interface utilisateur (54) qui affiche un indicateur d'état discret prédit du patient au personnel médical, l'indicateur d'état discret prédit étant une probabilité d'administration d'une intervention clinique pour la pluralité de segments de données stockés satisfaisant à des critères de similarité à seuil par rapport au segment de données patient ; et
un processeur matériel (40) qui est configuré pour exécuter le code logiciel de prédiction d'état physiologique pour :
réaliser une analyse de forme d'onde des données hémodynamiques afin de déterminer une pluralité de paramètres de profilage (53),
les paramètres de profilage comportant un ou plusieurs paramètres parmi une pression artérielle systolique, une pression artérielle diastolique, une pression artérielle moyenne standard, un volume d'éjection systolique, une fréquence cardiaque, une respiration, une contractilité cardiaque, des mesures de sensibilité baroréflexe, des mesures de complexité hémodynamique et des caractéristiques hémodynamiques dans le domaine fréquentiel ;
extraire un segment de données patient comprenant un ensemble de données patient pour un premier paramètre de profilage de la pluralité de paramètres de profilage ;
comparer le segment de données patient à une pluralité de segments de données stockés provenant d'une table de correspondance (52) dans une base de données, chacun de la pluralité de segments de données stockés présentant un ensemble de données d'état discret associé qui indique, pour chaque point temporel du segment de données stocké, si une intervention clinique a été ou non administrée et un type d'intervention clinique si elle a été administrée, et un ensemble de données pour le premier paramètre de profilage ; et
identifier une pluralité de segments de données stockés satisfaisant à des critères de similarité à seuil par rapport au segment de données patient.

8. Système selon la revendication 7, dans lequel le segment de données patient et le segment de données stocké comprennent chacun une période de temps fixe sur laquelle le premier paramètre de profilage est déterminé pour une pluralité de points temporels pris à intervalles réguliers afin de produire l'ensemble de données patient pour le premier paramètre de profilage et l'ensemble de données stocké pour le premier paramètre de profilage.

9. Système selon la revendication 7 ou la revendication 8, dans lequel le segment de données patient comprend en outre un état discret associé indiquant si une intervention clinique a été ou non administrée pendant le segment de données patient.

10. Système selon l'une quelconque des revendications précédentes, dans lequel la pluralité de segments de données stockés satisfaisant à des critères de similarité à seuil par rapport au segment de données patient sont identifiés par calcul d'une distance entre le segment de données patient et chacun de la pluralité de segments de données stockés au moyen d'une métrique de distance.

11. Système selon l'une quelconque des revendications précédentes, dans lequel la pluralité de segments de données stockés comprennent des segments de données obtenus à partir d'une pluralité de patients.

12. Système selon la revendication 11, et dans lequel la base de données comporte un classificateur de patient, le classificateur de patient comportant l'âge, une tranche d'âge, le sexe, une maladie ou une comorbidité du patient,
facultativement, dans lequel chaque segment de données stocké de la pluralité de segments de données stockés présente un classificateur de patient stocké associé.

13. Système d'entraînement d'un modèle prédictif afin de prédire un état physiologique indiquant si une intervention clinique a été ou non administrée sur la base d'une pression artérielle monitorée d'un patient, le système comprenant :
une mémoire système qui stocke un code logiciel d'entraînement de modèle pour le modèle prédictif ;
un processeur matériel configuré pour exécuter le code logiciel d'entraînement de modèle pour :
recevoir des données hémodynamiques représentant une forme d'onde de pression artérielle d'une population de patients en soins intensifs et comportant des étiquettes de données indiquant un type d'intervention clinique et un moment approximatif d'administration de l'intervention clinique ;
diviser les données hémodynamiques de la population de patients en soins intensifs en :
une sous-population de patients d'entraînement ; et
une sous-population de patients de validation ;
transformer les données hémodynamiques en une pluralité de paramètres de profilage caractérisant les données hémodynamiques, les paramètres de profilage comportant un ou plusieurs paramètres parmi une pression artérielle systolique, une pression artérielle diastolique, une pression artérielle moyenne standard, un volume d'éjection systolique, une fréquence cardiaque, une respiration, une contractilité cardiaque, des mesures de sensibilité baroréflexe, des mesures de complexité hémodynamique et des caractéristiques hémodynamiques dans le domaine fréquentiel ;
extraire une pluralité de segments de données à partir de données hémodynamiques pour chaque patient de la sous-population de patients d'entraînement et de la sous-population de patients de validation, chaque segment de données de la pluralité de segments de données représentant une période de temps fixe et comprenant (a) des points de données déterminés pour un paramètre de profilage sur la période de temps fixe et (b) les étiquettes de données indiquant une intervention en soins intensifs si elle a été administrée pendant la période de temps fixe ;
comparer des segments de données extraits à partir de la sous-population de patients de validation à des segments de données extraits à partir de la sous-population de patients d'entraînement au moyen d'une métrique de différence afin d'identifier des segments de données satisfaisant à des critères de similarité à seuil ; et
calculer une probabilité d'administration d'une intervention en soins intensifs pour chaque segment de données de la pluralité de segments de données extraits à partir de la sous-population de patients de validation.

14. Système selon la revendication 13, dans lequel la probabilité d'administration d'une intervention en soins intensifs est calculée par détermination (a) d'une probabilité initiale d'administration d'une ou de plusieurs interventions en soins intensifs pour les segments de données similaires, et (b) d'une probabilité de transition :
d'une intervention en soins intensifs vers une autre intervention en soins intensifs d'un type différent ;
d'aucune intervention en soins intensifs vers une intervention en soins intensifs ; ou
d'une intervention en soins intensifs vers aucune intervention en soins intensifs.

15. Système selon la revendication 14, dans lequel :
a) chaque segment de données extrait à partir de la sous-population de patients de validation est comparé à chaque segment de données extrait à partir de la sous-population de patients d'entraînement pour chaque patient de la sous-population de patients d'entraînement et chaque patient de la sous-population de patients de validation ; ou
b) le processeur matériel est en outre configuré pour comparer la probabilité calculée d'administration d'une intervention en soins intensifs pour chaque segment de données de la pluralité de segments de données extraits à partir de la sous-population de patients de validation aux étiquettes de données comprises dans chaque segment de données.
